# DEMANDE DE BREVET EUROPEEN

(11) **EP 4 710 753 A1**
(43) Date de publication de la demande: **18.03.2026**
(21) Numéro de dépôt: 25202066.4
(22) Date de dépôt: 15.09.2025
(51) Int. Cl.: A01G 9/24, A01G 17/00, A01H 1/02

(54) **PROCÉDÉ DE POLLINISATION**

(30) Priorité: 17.09.2024 FR 2409882
(71) Demandeur: Sun'Agri, 75017 Paris (FR)
(72) Inventeur: LOPEZ VELASCO, Gerardo, 34080 Montpellier (FR); FUMEY, Damien, 34070 Montpellier (FR)
(74) Mandataire: Ipsilon

(57) **Abrégé**

Procédé de pollinisation de végétaux (A), notamment d'arbres fruitiers, situés sous des capteurs photovoltaïques orientables (C), l'ombre projetée sur les végétaux étant modifiée par le changement d'orientation des capteurs, procédé dans lequel on agit automatiquement sur l'orientation des capteurs en fonction de l'état de floraison de manière à augmenter l'ensoleillement et/ou l'exposition au vent, par rapport à une référence de pilotage des capteurs appliquée lorsque la floraison est non mature ou non démarrée, pour encourager l'activité des pollinisateurs et/ou favoriser l'action du vent sur la pollinisation.

## Description

### Domaine technique

La présente invention concerne les procédé de pollinisation.

### Technique antérieure

La pollinisation est un mécanisme indispensable à la production de fruits pour une grande majorité de végétaux à fleurs de la planète. Ce processus consiste à transférer le pollen depuis l'appareil reproducteur mâle vers l'organe femelle de la fleur. Des vecteurs de pollen, comme le vent et certains insectes, sont donc nécessaires pour mener à bien cette opération.

La demande CN113424741 concerne un procédé de culture de kiwis sous une centrale photovoltaïque comportant des panneaux photovoltaïques non orientables. Ces derniers protègent les plantations du soleil en été et empêchent l'eau de pluie de pénétrer dans les troncs, ce qui peut bloquer efficacement la propagation du chancre. Lors de la pollinisation, le côté sud de la même plante est naturellement pollinisé, tandis que du côté nord une pollinisation artificielle doit être mise en œuvre.

La demande CN116784130 divulgue une serre intégrant entre autres la production d'énergie photovoltaïque. La serre comporte un système de pollinisation à rails mobiles.

### Exposé de l'invention

Il existe un besoin pour perfectionner encore les méthodes de pollinisation de végétaux, notamment d'arbres fruitiers, situés sous des capteurs photovoltaïques, afin d'optimiser le processus de pollinisation tout en produisant de l'énergie électrique.

L'invention vise à répondre à cet objectif et a pour objet, selon l'un de ses aspects, un procédé de pollinisation de végétaux, notamment d'arbres fruitiers, situés sous des capteurs photovoltaïques orientables, l'ombre projetée sur les végétaux étant modifiée par le changement d'orientation des capteurs, procédé dans lequel on agit automatiquement sur l'orientation des capteurs en fonction de l'état de floraison de manière à augmenter l'ensoleillement et/ou l'exposition au vent, par rapport à une référence de pilotage des capteurs appliquée lorsque la floraison est non mature ou non démarrée, pour encourager l'activité des pollinisateurs et/ou favoriser l'action du vent sur la pollinisation.

On entend par « floraison mature », la présence de plus de 20 % de fleurs pollinisables parmi une population de fleurs du végétal se trouvant dans une zone observable par une caméra ou un opérateur humain, une fleur pollinisable étant dans un état d'épanouissement total ou partiel selon la nature de la fleur.

On entend par « floraison non démarrée », l'absence ou moins de 20 % de fleurs pollinisables parmi une population de fleurs du végétal se trouvant dans une zone observable par une caméra ou un opérateur humain.

L'invention permet ainsi de profiter du caractère orientable des capteurs photovoltaïques pour encourager l'activité des pollinisateurs et/ou favoriser l'action du vent sur la pollinisation lorsque la floraison est mature.

De préférence, l'orientation des capteurs photovoltaïques est pilotée à partir au moins de données représentatives des stades phénologiques des végétaux, notamment l'état de la floraison, les indicateurs de l'état de floraison comprenant en particulier la densité des fleurs et/ou leur taille et/ou leurs couleurs et/ou la taille des organes les constituant et/ou le nombre de fleurs écloses par rapport au nombre total de fleurs.

Le stade phénologique d'un végétal fait référence à la phase spécifique de son cycle de croissance et de développement. Il décrit où le végétal se situe dans son cycle biologique en fonction des conditions météorologiques, de la saison et d'autres facteurs environnementaux. Les stades phénologiques d'un végétal comprennent plusieurs étapes clés telles que la dormance, le germé, le développement végétatif et la floraison.

On agit préférentiellement sur l'orientation des capteurs tout en cherchant à atteindre un optimum maximisant la production d'énergie électrique par rapport à une référence sans combinaison avec les végétaux.

Le pilotage des capteurs s'effectue avantageusement en fonction de la température extérieure et/ou du jour de la semaine, notamment en vue de diminuer l'ombre générée par les capteurs à des heures où la demande en énergie électrique est moindre.

Dans un mode de réalisation, les capteurs photovoltaïques sont orientés de manière à générer le moins d'ombre possible sur les végétaux à polliniser en période de floraison mature, la moyenne de l'énergie lumineuse reçue par les végétaux et intégrée sur une journée étant notamment maximale durant les périodes de floraison mature.

Au moins une caméra peut être utilisée pour acquérir des images des végétaux et suivre l'état de floraison. Le procédé comporte notamment le traitement automatique des images de la caméra pour détecter le stade de la floraison et pour générer une information sur la base de laquelle les capteurs sont pilotés.

Les images prises par la caméra sont préférentiellement comparées à des images de référence indiquant le stade de la floraison.

Une segmentation des images prises par la caméra peut être opérée pour identifier les fleurs. Un traitement colorimétrique des images peut être effectué pour analyser les couleurs des fleurs. La dimension et/ou la couleur des organes de la fleur peut être utilisée comme indicateur pour déterminer le stade de la floraison.

De préférence, si les images prises par la caméra montrent au moins la présence de plus de 20% de fleurs prêtes à être pollinisées, alors la floraison est considérée comme mature et un mode de pilotage des capteurs favorisant la pollinisation est activé.

De préférence, l'état de floraison est périodiquement réévalué pendant la période de floraison, notamment tous les jours.

Dans un mode de réalisation, une application pour terminal mobile est mise à disposition d'au moins un utilisateur pour permettre d'entrer au moins une information relative à l'état de la floraison, cette information étant transmise à un système de pilotage des capteurs. De préférence, l'application est configurée pour géolocaliser automatiquement le terminal mobile, et le terminal transmet au moins une information relative à l'état de la floraison et/ou à l'activité des pollinisateurs à proximité du terminal, ainsi qu'une information sur la localisation du terminal.

On peut prendre au moins une photographie des végétaux avec le terminal mobile, et cette photographie peut être analysée automatiquement pour en déduire au moins une information concernant l'état de la floraison et/ou de l'activité des pollinisateurs, la photographie étant de préférence géolocalisée.

Dans un mode de réalisation, la présence de vent dans l'environnement des végétaux, notamment sa vitesse et sa direction, est détectée par un capteur de vent, notamment un anémomètre.

En période de floraison mature et en cas de vent dépassant une vitesse prédéfinie, les capteurs photovoltaïques sont de préférence orientés parallèlement à la pente du terrain.

Les végétaux peuvent être choisis parmi les arbres fruitiers, notamment les arbres fruitiers à pépins et à noyaux, en particulier les pommiers, poiriers, pruniers, abricotiers, figuiers, actinidias, cerisiers et pêchers.

L'invention a encore pour objet, selon un autre de ses aspects, un procédé de culture de végétaux sous capteurs photovoltaïques orientables, dans lequel on cultive ces végétaux tout en agissant sur l'activité de pollinisateurs des végétaux par la mise en œuvre du procédé de pollinisation selon l'invention.

### Brève description du dessin

L'invention pourra être mieux comprise à la lecture de la description détaillée qui va suivre, d'exemples non limitatifs de mise en œuvre de celle-ci, et à l'examen du dessin annexé, sur lequel :
[Fig 1] La figure 1 représente de façon schématique un système de commande de l'orientation d'un capteur photovoltaïque selon l'invention ;
[Fig 2] La figure 2 est une vue schématique d'un système de production d'énergie électrique comportant des capteurs photovoltaïques permettant la mise en œuvre du procédé de pollinisation selon l'invention ; et
[Fig 3] La figure 3 est analogue à la figure 2 avec prise en compte du vent comme acteur de pollinisation.

### Description détaillée

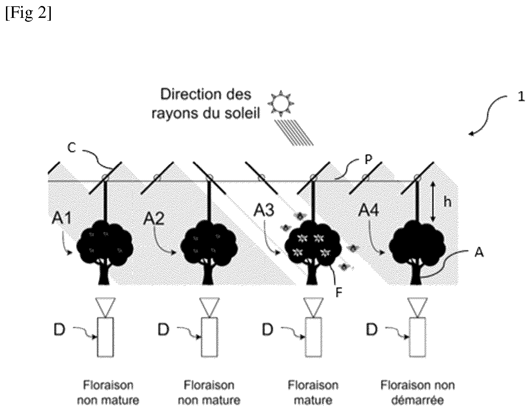

On a illustré schématiquement à la figure 2 un système de production d'énergie électrique 1, comportant une structure porteuse P et des capteurs photovoltaïques orientables C maintenus à distance non nulle du sol par la structure porteuse P, et à une hauteur h d'arbres fruitiers A, par exemple des pommiers, comportant des fleurs F, situés sous les capteurs C. L'ombre solaire projetée sur les végétaux est modifiée par le changement d'orientation des capteurs C.

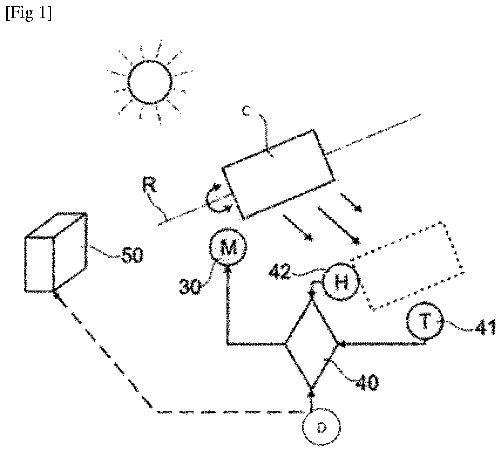

La figure 1 représente un de ces capteurs photovoltaïques C qui est mobile autour d'un axe de rotation R. Le capteur photovoltaïque C est entraîné autour de l'axe R à l'aide d'au moins un actuateur 30. Par exemple, il est prévu individuellement pour chaque capteur photovoltaïque C un actuateur 30. En variante, un même actuateur 30 peut déplacer en rotation une pluralité de capteurs photovoltaïques C.

Les actuateurs 30 comportent par exemple chacun un ou plusieurs moteurs électriques, et sont constitués par exemple par des servomoteurs.

La position à donner au capteur photovoltaïque C peut être déterminée par un calculateur local 40 qui est relié via toute interface de puissance adaptée à l'actuateur 30.

Le calculateur 40 reçoit de préférence des informations météorologiques, notamment d'une ou plusieurs sondes locales, par exemple une sonde de température 41 et une sonde d'hygrométrie 42. D'autres sondes peuvent être ajoutées pour scruter les conditions météorologiques, tels qu'un pluviomètre, anémomètre, et/ou une caméra D pour acquérir des images et visualiser l'état de développement du végétal et l'état de la floraison, ainsi qu'un ou plusieurs biocapteurs, le cas échéant. Ladite caméra D peut être soit portée par la structure P soit par une autre structure à part, soit par un drone.

Le calculateur 40 peut également échanger des données, par exemple via un réseau de téléphonie sans fil, avec un serveur distant 50, lequel peut par exemple informer le calculateur 40 de la météo à venir. Les données locales de température et de vent peuvent ainsi provenir d'un serveur météo distant.

Le calculateur 40 peut être réalisé à partir de tout micro-ordinateur ou équipement informatique permettant de piloter l'orientation des capteurs photovoltaïques C en fonction d'une ou plusieurs lois de commande donnant l'orientation à imposer aux capteurs photovoltaïques en fonction de données représentatives des stades phénologiques des végétaux, notamment l'état de la floraison, les indicateurs de l'état de la floraison comprenant en particulier la densité des fleurs F et/ou leur taille et/ou leurs couleurs et/ou la taille des organes les constituant et/ou le nombre de fleurs écloses par rapport au nombre total de fleurs. L'orientation à imposer aux capteurs photovoltaïques C peut aussi être commandée selon la variété du végétal, les conditions météorologiques, l'activité des pollinisateurs et/ou l'état de la floraison. Le calculateur 40 peut être agencé pour traiter des images délivrées par la caméra D afin de déterminer l'état de la floraison. Alternativement, un opérateur humain observe l'état de floraison, par exemple au niveau d'une branche témoin, et dans le cas où le niveau de floraison correspond à l'existence de la population souhaitée de fleurs pollinisables, signale la maturité de la floraison à l'aide d'une application mobile connectée au calculateur 40.

Le calculateur 40 peut comporter une unité de calcul et une mémoire locale dans laquelle peuvent être enregistrées des données locales relatives aux végétaux et/ou à leur environnement.

La mémoire du calculateur peut également comporter des paramètres d'asservissement qui régissent l'orientation des capteurs photovoltaïques C en fonction d'objectifs de pollinisation. Ces paramètres peuvent évoluer dans le temps et, en fonction par exemple de la saison, et privilégier la pollinisation ou non des végétaux.

La ou les lois de commande peuvent être initialement programmées dans le calculateur 40, ou en variante être téléchargées par le calculateur 40 à partir du serveur distant 50, ou encore être réactualisées périodiquement par le serveur distant 50.

Dans un exemple de réalisation, le calculateur 40 présente un fonctionnement autonome. En fonction de la saison, de la date de semis, du nombre fleurs présents et/ou de l'état de la floraison, et éventuellement d'autres paramètres renseignés par l'agriculteur, il pilote automatiquement et de façon journalière ou avec une autre périodicité l'orientation des capteurs photovoltaïques C de façon à atteindre l'objectif de pollinisation sur une période donnée.

Le calculateur 40 est agencé pour traiter les images acquises par la caméra D et identifier l'état de la floraison par la comparaison de ces images à des images de référence. Une segmentation des images acquises peut être opérée pour identifier les fleurs. Un traitement colorimétrique des images peut être effectué pour analyser les couleurs des fleurs. La dimension et/ou la couleur des organes de la fleur peut être utilisée comme indicateur pour déterminer le stade de la floraison.

En fonction de l'état de la floraison, les capteurs C sont orientés soit pour projeter le maximum d'ombre sur les arbres A, soit pour minimiser l'ombre portée en laissant passer la lumière et favoriser ainsi l'activité des pollinisateurs, en l'occurrence des insectes.

Par exemple, si les images prises par la caméra D montrent la présence de la population requise de fleurs prêtes à être pollinisées, alors la floraison est considérée mature et un mode de pilotage des capteurs favorisant la pollinisation est activé.

Lorsque ce mode de pilotage est activé, les capteurs photovoltaïques C sont par exemple orientés pendant plusieurs jours pour favoriser le plus possible le passage de la lumière et l'action des pollinisateurs tels que des insectes, notamment des abeilles. Puis, une fois la floraison passée, les capteurs photovoltaïques C sont par exemple pilotés en activant les actuateurs 30 pour prendre une orientation visant à laisser passer le minimum de lumière, maximisant la production électrique.

On voit sur la figure 2 que, lorsque la floraison est mature, les capteurs C sont orientés selon la direction des rayons du soleil, projetant ainsi le minimum d'ombre sur l'arbre A3. Au contraire, lorsque la floraison est encore non mature ou non démarrée, les capteurs C sont orientés de manière à porter le maximum d'ombre sur les arbres A1, A2 et A4.

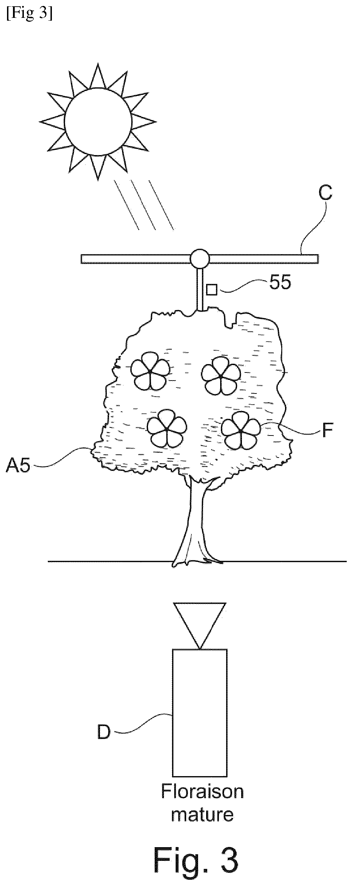

La figure 3 montre un arbre A5 en état de floraison mature. L'installation reçoit une information sur la vitesse du vent d'un serveur météo et/ou comporte un anémomètre 55 qui fournit cette information.

Lorsque la vitesse du vent convient à la pollinisation, le capteur C est orienté à l'horizontale de manière à laisser passer le maximum de vent sous les panneaux pour favoriser la pollinisation des fleurs.

## Revendications

1. Procédé de pollinisation de végétaux (A), notamment d'arbres fruitiers, situés sous des capteurs photovoltaïques orientables (C), l'ombre projetée sur les végétaux étant modifiée par le changement d'orientation des capteurs, procédé dans lequel on agit automatiquement sur l'orientation des capteurs en fonction de l'état de floraison de manière à augmenter l'ensoleillement et/ou l'exposition au vent, par rapport à une référence de pilotage des capteurs appliquée lorsque la floraison est non mature ou non démarrée, pour encourager l'activité des pollinisateurs et/ou favoriser l'action du vent sur la pollinisation.

2. Procédé selon la revendication précédente, l'orientation des capteurs photovoltaïques (C) étant pilotée à partir au moins de données représentatives des stades phénologiques des végétaux (A), notamment l'état de la floraison, les indicateurs de l'état de la floraison comprenant en particulier la densité des fleurs (F) et/ou leur taille et/ou leurs couleurs et/ou la taille des organes les constituant et/ou le nombre de fleurs écloses par rapport au nombre total de fleurs.

3. Procédé selon l'une des deux revendications précédentes, dans lequel on agit sur l'orientation des capteurs (C) tout en cherchant à atteindre un optimum maximisant la production d'énergie électrique par rapport à une référence sans combinaison avec les végétaux (A).

4. Procédé selon l'une quelconque des revendications précédentes, dans lequel le pilotage des capteurs (C) s'effectue en fonction de la température extérieure et/ou du jour de la semaine, notamment en vue de diminuer l'ombre générée par les capteurs à des heures où la demande en énergie électrique est moindre.

5. Procédé selon l'une quelconque des revendications précédentes, les capteurs photovoltaïques (C) étant orientés de manière à générer le moins d'ombre possible sur les végétaux (A) à polliniser en période de floraison mature, la moyenne de l'énergie lumineuse reçue par les végétaux et intégrée sur une journée étant notamment maximale durant les périodes de floraison mature.

6. Procédé selon l'une quelconque des revendications précédentes, au moins une caméra (D) étant utilisée pour acquérir des images des végétaux (A) et suivre l'état de floraison, le procédé comportant notamment le traitement automatique des images de la caméra (D) pour détecter le stade de la floraison et pour générer une information sur la base de laquelle les capteurs (C) sont pilotés.

7. Procédé selon la revendication précédente, les images prises par la caméra (D) étant comparées à des images de référence indiquant le stade de la floraison.

8. Procédé selon l'une des deux revendications précédentes, une segmentation des images prises par la caméra (D) étant opérée pour identifier les fleurs (F).

9. Procédé selon l'une quelconque des revendications 6 à 8, un traitement colorimétrique des images prises par la caméra (D) étant effectué pour analyser les couleurs des fleurs (F).

10. Procédé selon l'une quelconque des revendications 6 à 9, dans lequel si les images prises par la caméra (D) montrent la présence de plus de 20% de fleurs (F) prêtes à être pollinisées, alors la floraison est considérée comme mature et un mode de pilotage des capteurs favorisant la pollinisation est activé.

11. Procédé selon l'une quelconque des revendications précédentes, dans lequel l'état de floraison est périodiquement réévalué pendant la période de floraison, notamment tous les jours.

12. Procédé selon l'une quelconque des revendications précédentes, dans lequel une application pour terminal mobile est mise à disposition d'au moins un utilisateur pour permettre d'entrer au moins une information relative à l'état de floraison, cette information étant transmise à un système de pilotage des capteurs (C).

13. Procédé selon la revendication précédente, dans lequel l'application est configurée pour géolocaliser automatiquement le terminal mobile, et dans lequel le terminal transmet au moins une information relative à l'état de la floraison et/ou à l'activité des pollinisateurs à proximité du terminal, ainsi qu'une information sur la localisation du terminal.

14. Procédé selon l'une des deux revendications précédentes, dans lequel on prend au moins une photographie des végétaux (A) avec le terminal mobile, et dans lequel cette photographie est analysée automatiquement pour en déduire au moins une information concernant l'état de la floraison et/ou de l'activité des pollinisateurs, la photographie étant de préférence géolocalisée.

15. Procédé selon l'une quelconque des revendications précédentes, dans lequel la présence de vent dans l'environnement des végétaux (A), notamment sa vitesse et sa direction, est détectée par un capteur de vent (55), notamment un anémomètre.

16. Procédé selon la revendication précédente, dans lequel en période de floraison mature et en cas de vent dépassant une vitesse prédéfinie, les capteurs photovoltaïques (C) sont orientés parallèlement à la pente du terrain.

17. Procédé selon l'une quelconque des revendications précédentes, les végétaux (A) étant choisis parmi les arbres fruitiers, notamment les arbres fruitiers à pépins et à noyaux, en particulier les pommiers, poiriers, pruniers, abricotiers, figuiers, actinidias, cerisiers et pêchers.

18. Procédé de culture de végétaux sous capteurs photovoltaïques orientables, dans lequel on cultive ces végétaux tout en agissant sur l'activité de pollinisateurs des végétaux par la mise en œuvre du procédé de pollinisation selon l'une quelconque des revendications 1 à 17.
